(19) Europäisches Patentamt
European Patent Office
Office européen des brevets

(11) EP 0 689 844 A1

(12) EUROPEAN PATENT APPLICATION

(43) Date of publication:
03.01.1996 Bulletin 1996/01

(51) Int. Cl.$^6$: A61K 47/48

(21) Application number: 94670004.4

(22) Date of filing: 23.06.1994

(84) Designated Contracting States:
AT BE CH DE DK ES FR GB GR IE IT LI LU MC NL PT SE

(71) Applicant: TECNIMEDE-SOCIEDADE TECNICO-MEDICINAL, S.A.
P-2685 Sacavém (PT)

(72) Inventor: De Sousa Goucha Jorge, Pedro Manuel
Lisboa (PT)

(74) Representative: Pereira da Cruz, Joao
P-1200 Lisboa (PT)

Remarks:
Amended claims in accordance with Rule 86 (2) EPC.

# (54) Complexes of vinpocetine formed with cyclodextrins, process for their preparation and pharmaceutical compositions containing them

(57) The present invention relates to pharmaceutical compositions, useful in the treatment of cerebrovascular disorders, containing an inclusion complex of vinpocetine formed with any kind of cyclodextrin. More specifically, the present invention relates to an inclusion complex of vinpocetine formed with any kind of cyclodextrin, preferably α-cyclodextrin, β-cyclodextrin, γ-cyclodextrin and hydroxypropyl-β-cyclodextrin, a process for its preparation, a pharmaceutical composition containing said complex and a process for preparing said composition.

EP 0 689 844 A1

## Description

### Field of the Invention

The present invention relates to pharmaceutical compositions, useful in the treatment of cerebrovascular disorders, containing an inclusion complex of vinpocetine (eburnamenine-14-carboxylic acid ethyl ester) formed with any kind of cyclodextrin.

### Background of the Invention

Cyclodextrins, also called Schardinger dextrins, cycloamyloses, cyclomaltoses and cycloglucans, are cyclic oligosaccharides. There exists three different kinds of cyclodextrins, i.e. the α-, β- and γ-cyclodextrin which consists of 6, 7 or 8 glucopiranose units connected with α-1,4 glucosidic bonds. The three cyclodextrins differ from each other in molecular weight, water solubility and in hollow-diameter, accordingly they are able to form inclusion complex with the most different kinds of compounds. There is a possibility to carry out further modifications in the cyclodextrin molecule with suitable substitutions. The solubility of these compounds is even more better and their complex-forming capacity also differs from that of the unsubstituted cyclodextrin.

It is generally characteristic for the cyclodextrins that the external surface of the molecule is hydrophilic but the inside of the cavity is hydrophobic in character. Because of these characteristics cyclodextrins are able to include hydrophobic molecules.

It is known that complexes of poor soluble active ingredients formed with cyclodextrins can dissolve in water easily. Their solubility may be 10 to 1,000-fold higher than that of the free, not-complexed active ingredients.

### Detailed Description of the Invention

Vinpocetine has the formula:

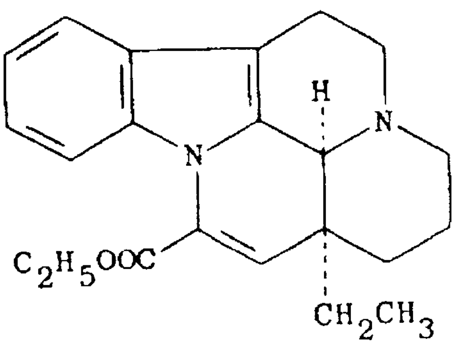

and has the following characteristics: molecular weight: 350.43 D; melting point: 149-153°C; it is poorly soluble in water, ethyl ether and hexane but dissolves well in ethanol and 0.1N HCl.

Vinpocetine is used for the treatment of cerebrovascular disorders and is usually available in tablets containing 5 mg of active ingredient. However, existing formulations exhibit poor bioavailability due to its reduced solubility and absorption. In fact, it has been observed that absolute bioavailability of vinpocetine administered p.o. is only 6.7%. Therefore, the therapeutical effects are not according to expectations.

To overcome these difficulties with substances having a very poor water solubility there has been used complexing with cyclodextrins.

Vinpocetine has a structure which allows at least a portion of the molecule to be housed inside the cavity of the cyclodextrin. One of the evidence of the formation of the complex between vinpocetine and cyclodextrin is that cyclodextrin increases the solubility of vinpocetine in aqueous solutions.

Thus, it is a first object of the invention an inclusion complex of vinpocetine formed with any kind of cyclodextrin, preferably α-cyclodextrin, β-cyclodextrin, γ-cyclodextrin and hydroxypropyl-β-cyclodextrin.

It is a second object of the invention a process for preparing an inclusion complex of vinpocetine formed with any kind of cyclodextrin, which comprises reacting vinpocetine with the cyclodextrin in the presence of an aqueous or aqueous-organic solvent medium.

It is a third object of the invention a pharmaceutical composition which comprises said inclusion complex of vinpocetine formed with any kind of cyclodextrin, as active substance, together with a pharmaceutically acceptable inert carrier.

We found that the *in vitro* solubility of tablets containing complexed vinpocetine is almost 10-fold higher as compared to that of not-complexed vinpocetine.

These compositions have a cerebrovascular vasodilatatory effect and contain a therapeutically effective amount of an inclusion complex of vinpocetine with cyclodextrin.

Suitable forms of pharmaceutical compositions are dosage forms, such as tablets or capsules, with an instant release or modified release of the active substance. These formulations may also contain, in addition to the active substance, the usual non-toxic inert solid or liquid carriers and/or additive materials which are commonly used in these compositions and are useful for enteral administration.

Preferably, the inclusion complex is dispersed in a polymer matrix that provides modified release of the active ingredient or is dispersed onto inert microspheres and coated with a rate-controlling polymer.

The polymer is, usually, made of cellulose derivatives or is Eudragit.

The pharmaceutical compositions according to the invention contain 1.0 to 50.0 mg, preferably 5.0 to 20.0 mg of active ingredient. The compositions preferably are finished as formulations suitable for enteral administration.

It is a fourth object of the invention a process for the preparation of the above pharmaceutical composition, which comprises mixing said complex of vinpocetine formed with any kind of cyclodextrin, as active substance, with pharmaceutically acceptable inert carriers and/or additive materials.

The pharmaceutical compositions are prepared by using the well-known methods such as stirring, sieving, mixing, granulating, pressing, etc. The compositions may be subjected to further operations used in the pharmaceutical industry.

According to another aspect of the invention there are provided processes for the preparation of modified release formulations. Vinpocetine exhibits properties that justify its oral administration as extended release formulation: rapid absorption after dissolution in the gastrointestinal (GI) tract, short elimination half-life and use in long term therapeutics.

## Brief Description of the Drawings

Figure 1 shows a phase solubility diagram of vinpocetine/cyd in water, concerning the complexes between vinpocetine and hydroxypropyl-β-cyclodextrin and between vinpocetine and γ-cyclodextrin.

Figure 2 shows thermograms obtained by differential scanning calorimetry (DSC) concerning several vinpocetine/cyd systems.

Figure 3 shows dissolution profiles of vinpocetine/β-cyclodextrin tablets.

The present invention is further illustrated by the aid of the following non-limiting Examples.

## EXAMPLES

### Example referring to the solubility of vinpocetine in cyclodextrin solutions of different concentrations

### EXAMPLE 1

10 mg of vinpocetine, cyclodextrin in different amounts and 10 ml of distilled water were charged into 20 ml test tubes. After closing, the tubes were agitated for 24 hours at 37°C, thereafter the samples were filtrated and the filtrates were analysed by HPLC (High Performance Liquid Chromatography) in 0.1N HCl solution. The results are shown in Table I and Fig. 1.

TABLE I

| Cyclodextrin (mM) | Solubility of vinpocetine in μg/ml determined at 37°C in cyclodextrin solutions of different concentration | |
|---|---|---|
| | HP-β-cyd | γ-cyd |
| 12.5 | --- | 6.1 |
| 25.0 | 5.5 | 12.0 |
| 50.0 | 12.8 | 24.4 |
| 100.0 | 23.2 | --- |

**Examples for the preparation of vinpocetine-β-cyclodextrin complexes having a molar ratio of 1:2**

**EXAMPLE 2**

**Preparation of the complex according to the spray-drying method**

900 mg (793 μmol) of β-cyclodextrin were dissolved in 50 ml of 0.02N HCl at 25°C. 138.95 mg (396.5 μmol) of vinpocetine were added to this solution and dissolved under vigorous stirring. The solution was then neutralized with 8.4 ml of 0.1N NaOH. After a further 16-hours intensive stirring at 25°C, the solution was filtered through a 0.45 μm PE membrane. The filtrate was spray-dried at 70°C to give 335.2 mg of product that contains 12.2% by weight of vinpocetine and a molar ratio of vinpocetine to β-Cyclodextrin = 1:2.2.

**Preparation of complexes according to the co-precipitation method**

**EXAMPLE 3**

1,297 mg of β-cyclodextrin (1.143 mmol) were dissolved in 50 ml of water at 40°C and a solution containing 200 mg of vinpocetine (0.571 mmol) in 10 ml of 0.1N HCl was added under stirring. The mixture was then heated to a temperature of 60°C and stirred for 2 hours at this temperature. The obtained solution was evaporated under reduced pressure at 40°C to a dry product.

The thermogram obtained by differential scanning calorimetry (DSC) of the microcrystalline product obtained shows no endothermic peak around the melting temperature of vinpocetine (Fig.2, Curve 2).

**EXAMPLE 4**

1,297 mg of β-cyclodextrin (1.143 mmol) were dissolved in 50 ml of water at 40°C and a solution containing 200 mg of vinpocetine (0.571 mmol) in 10 ml of 2% (w/v) tartaric acid solution was added under stirring. The mixture was then heated to a temperature of 60°C and stirred for 2 hours at this temperature. The obtained solution was evaporated under reduced pressure at 40°C to a dry product.

The thermogram obtained by differential scanning calorimetry (DSC) of the microcrystalline product obtained shows no endothermic peak around the melting temperature of vinpocetine (Fig. 2, Curve 3).

**EXAMPLE 5**

**Preparation of the complex according to the kneading method**

2,594 mg of β-cyclodextrin (2.285 mmol) and 400 mg of vinpocetine (1.142 mmol) were triturated with 1 ml of a 17% (w/v) tartaric acid solution and the slurry was kneaded thoroughly for about 30 minutes. The resulting paste was dried under reduced pressure at 50°C for 8 hours.

The thermogram obtained by differential scanning calorimetry (DSC) of the complex shows no endothermic peak around the melting temperature of vinpocetine (Fig. 2, Curve 4).

**Examples of pharmaceutical compositions containing vinpocetine-cyclodextrin complex as active ingredient**

**EXAMPLE 6**

**Tablets containing 10 mg of vinpocetine in form of HP-β-cyclodextrin complex**

The dissolution properties of tablets containing 10 mg of vinpocetine (tablet A) and tablets containing 10 mg of vinpocetine in form of HP-β-cyclodextrin complex prepared according to the kneading method (tablet B) were compared at 37°C in 1,000 ml of water using apparatus 2 of USP XXII (paddle method). One tablet was placed into each vessel and the amount of the dissolved vinpocetine was determined by HPLC.

The compositions of the tested tablets were the following:

Tablet A:

| | |
|---|---|
| Vinpocetine | 10.0 mg |
| Lactose | 227.6 mg |
| Microcrystalline cellulose | 27.0 mg |
| Magnesium stearate | 5.4 mg |
| | 270.0 mg |

Tablet B:

| | |
|---|---|
| Complex containing 10 mg of vinpocetine | 193.8 mg |
| Croscarmellose sodium | 4.0 mg |
| Aerosil 200 | 0.2 mg |
| Magnesium stearate | 2.0 mg |
| | 200.0 mg |

The results of the dissolution test are summarized in table II.

TABLE II

| **Time (min)** | **Amounts of the dissolved active ingredient (mg)** | |
|---|---|---|
| | **Tablet A** | **Tablet B** |
| 30 | 0.72 | 5.16 |
| 60 | 1.64 | 7.16 |
| 120 | 2.59 | 8.98 |

The results show that vinpocetine dissolves very slightly from the conventional tablets but in case of a tablet containing the same amount of vinpocetine in form of HP-β-cyclodextrin complex, the dissolved quantity of the active ingredient after 15 minutes is 7-fold higher than the value obtained by the conventional tablets.

**EXAMPLE 7**

**Extended release matrix tablets containing 10 mg of vinpocetine in form of β-cyclodextrin complex**

Matrix tablets containing 10 mg of vinpocetine in form of β-cyclodextrin complex (prepared according to example 5) were formulated as follows:

| | **Methocel K4M (%)** | | |
|---|---|---|---|
| | **16** | **20** | **24** |
| Vinpocetine | 10.0 mg | 10.0 mg | 10.0 mg |
| β-cyclodextrin | 65.0 mg | 65.0 mg | 65.0 mg |
| Methocel K4MP | 16.4 mg | 21.5 mg | 27.2 mg |
| Tartaric acid | 10.0 mg | 10.0 mg | 10.0 mg |
| Magnesium stearate | 1.0 mg | 1.0 m | 1.0 mg |
| | 102.4 mg | 107.5 mg | 113.2 mg |

Release profiles of the tablets were determined by dissolution assays in water at 37°C ± 1°C using apparatus 2 of USP XXII. The *in vitro* dissolution data are shown in Table III and Fig.3.

TABLE III

| **Time (min)** | **Methocel K4M (%)** | | |
|---|---|---|---|
| | **16** | **20** | **24** |
| | **% released** | | |
| 30 | 33.4 | 29.0 | 21.8 |
| 60 | 48.4 | 42.1 | 32.7 |
| 120 | 70.2 | 61.9 | 50.3 |
| 180 | 84.2 | 76.1 | 63.3 |
| 240 | 93.6 | 87.6 | 74.7 |
| 360 | 99.6 | 102.6 | 92.2 |
| 480 | 101.4 | 105.0 | 100.5 |

## Claims

1. Inclusion complex of vinpocetine formed with any kind of cyclodextrin.

2. Inclusion complex of vinpocetine according to claim 1 wherein the cyclodextrin is α-cyclodextrin.

3. Inclusion complex of vinpocetine according to claim 1 wherein the cyclodextrin is β-cyclodextrin.

4. Inclusion complex of vinpocetine according to claim 1 wherein the cyclodextrin is γ-cyclodextrin.

5. Inclusion complex of vinpocetine according to claim 1 wherein the cyclodextrin is hydroxypropyl-β-cyclodextrin.

6. Process for preparing an inclusion complex of vinpocetine formed with any kind of cyclodextrin, which comprises reacting vinpocetine with the cyclodextrin in the presence of an aqueous or aqueous-organic solvent medium.

7. Pharmaceutical composition which comprises the inclusion complex of vinpocetine formed with any kind of cyclodextrin as defined in any one of claims 1 to 5, as active substance, together with a pharmaceutically acceptable inert carrier.

8. Pharmaceutical composition according to claim 7 wherein the inclusion complex is dispersed in a polymer matrix that provides modified release of the active ingredient.

9. Pharmaceutical composition according to claim 7 wherein the inclusion complex is dispersed onto inert spheres and coated with a rate-controlling polymer.

10. Pharmaceutical composition according to any one of claims 8 and 9 wherein the polymer comprises cellulose derivatives.

11. Pharmaceutical composition according to any one of claims 8 and 9 wherein the polymer is Eudragit.

12. Pharmaceutical composition according to any one of claims 7, 8 and 9 wherein the administration form is a tablet or a capsule with an instant release or modified release of the active substance together with the usual non-toxic inert solid or liquid carriers and/or additive materials which are commonly used in these compositions and are useful for enteral administration.

13. Pharmaceutical composition according to any one of claims 7 to 12 wherein the amount of active substance ranges from 1.0 to 50.0 mg.

14. Pharmaceutical composition according to any one of claims 7 to 13 for the treatment of cerebrovascular disorders.

15. Process for the preparation of a pharmaceutical composition, as defined in any one of the claims 7 to 14, which comprises mixing a complex of vinpocetine formed with any kind of cyclodextrin, as defined in any one of claims 1 to 5, as active substance, with pharmaceutically acceptable inert carriers and/or additive materials.

**Amended claims in accordance with Rule 86(2) EPC.**

1. Inclusion complex of vinpocetine formed with any kind of cyclodextrin.

2. Inclusion complex of vinpocetine according to claim 1 wherein the cyclodextrin is $\alpha$-cyclodextrin.

3. Inclusion complex of vinpocetine according to claim 1 wherein the cyclodextrin is $\beta$-cyclodextrin.

4. Inclusion complex of vinpocetine according to claim 1 wherein the cyclodextrin is hydroxypropyl-$\beta$-cyclodextrin.

5. Process for preparing an inclusion complex of vinpocetine formed with any kind of cyclodextrin, which comprises interacting vinpocetine with the cyclodextrin in the presence of an aqueous, aqueous-organic or organic solvent medium.

6. Pharmaceutical composition which comprises the inclusion complex of vinpocetine formed with any kind of cyclodextrin, as defined in any one of claims 1 to 4, as active substance, together with a pharmaceutically acceptable inert carrier.

7. Pharmaceutical composition according to claim 6 wherein the inclusion complex is dispersed in a polymer matrix that provides modified release of the active ingredient.

8. Pharmaceutical composition according to claim 6 wherein the inclusion complex is dispersed onto inert spheres and coated with a rate-controlling polymer.

9. Pharmaceutical composition according to any one of claims 7 and 8 wherein the polymer comprises cellulose derivatives.

10. Pharmaceutical composition according to any one of claims 7 and 8 wherein the polymer is Eudragit.

11. Pharmaceutical composition according to any one of claims 6, 7 and 8 wherein the administration form is a tablet or a capsule with an instant release or modified release of the active substance together with the usual non-toxic inert solid or liquid carriers and/or additive materials which are commonly used in these compositions and are used for oral administration.

12. Pharmaceutical composition according to any one of claims 6 to 11 wherein the amount of active substance ranges from 1.0 to 50.0 mg.

13. Pharmaceutical composition according to any one of claims 6 to 12 for the treatment of cerebrovascular disorders.

14. Process for the preparation of a pharmaceutical composition, as defined in any one of the claims 6 to 13, which comprises mixing a complex of vinpocetine formed with any kind of cyclodextrin, as defined in any one of claims 1 to 4, as active substance, with pharmaceutically acceptable inert carriers and/or additive materials.

# Phase Solubility Diagram of Vinpocetine/Cyd in Water at 37°C

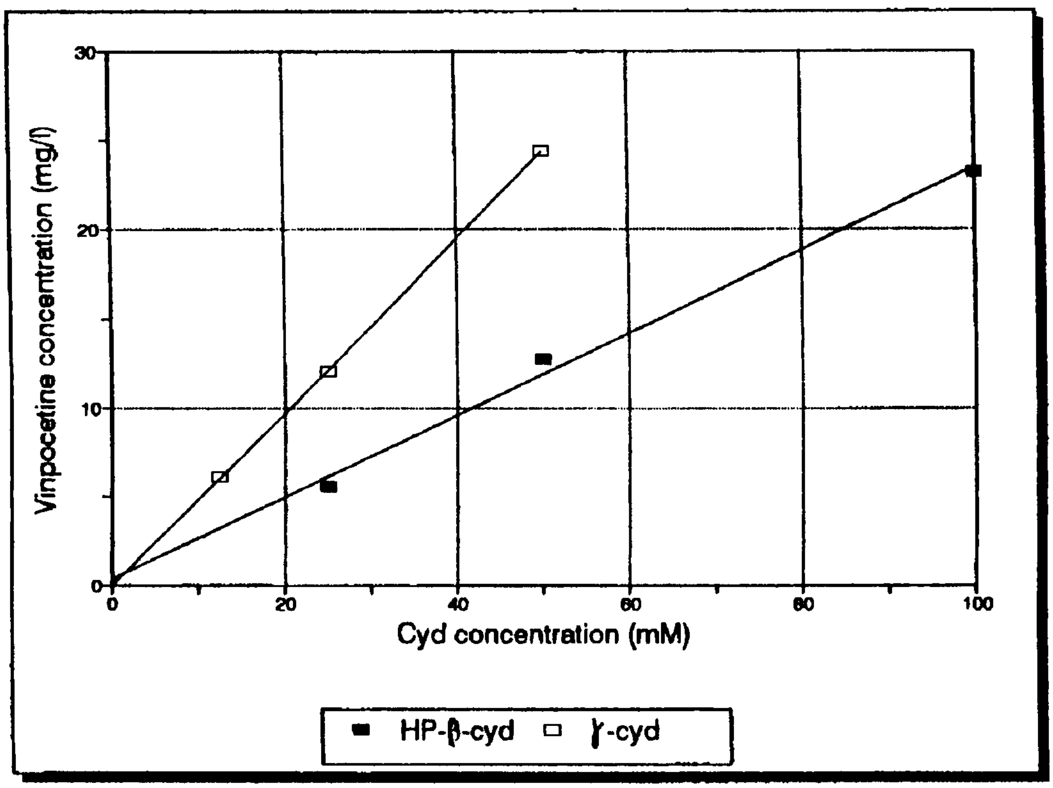


Fig. 1

# DSC Thermograms of Vinpocetine/Cyd Systems

*1) Vinpocetine*
*2) Complex according to Example 3*
*3) Complex according to Example 4*
*4) Complex according to Example 5*

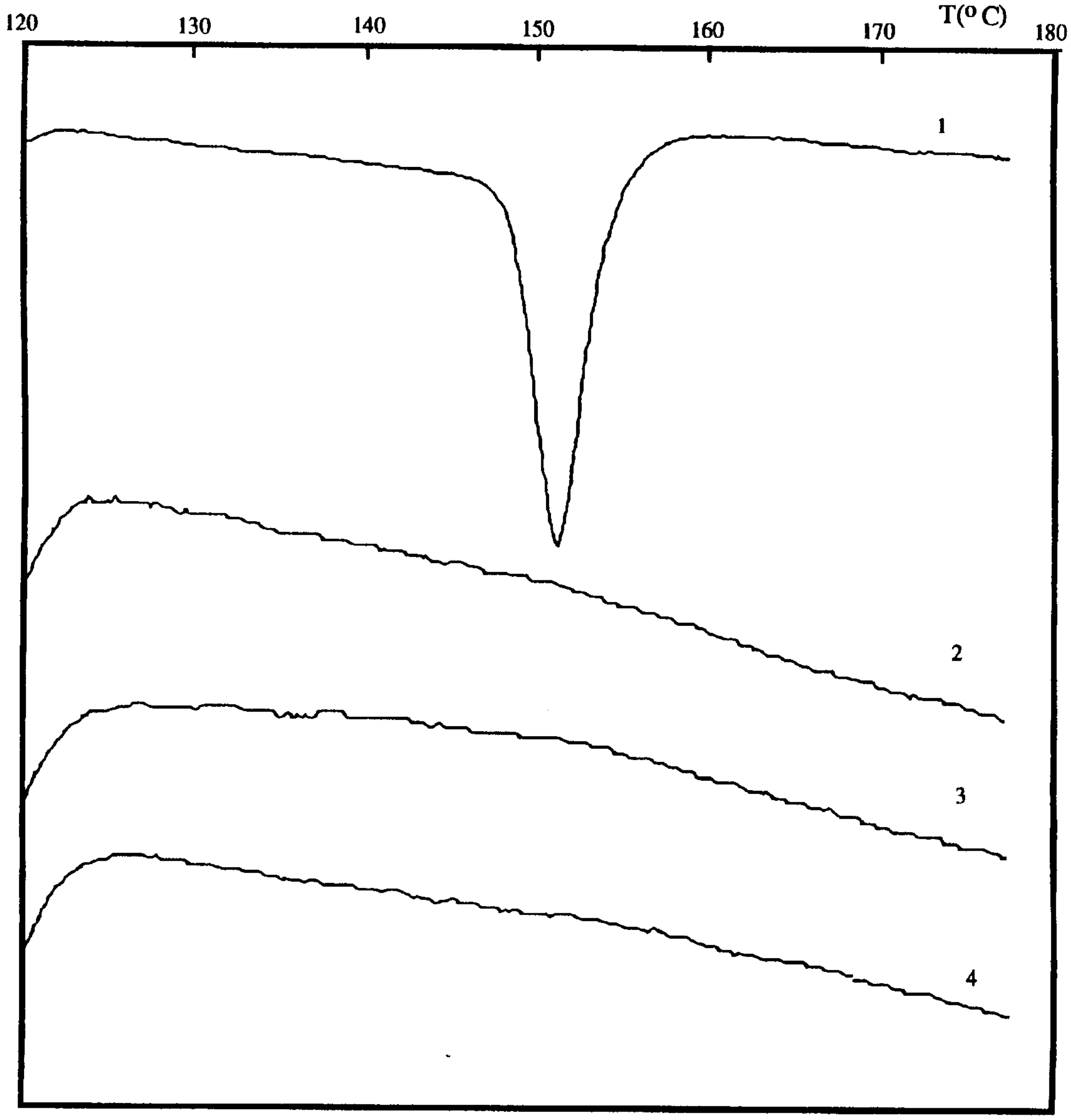


**Fig. 2**

# Dissolution profiles of extended release tablets prepared according to Example 7

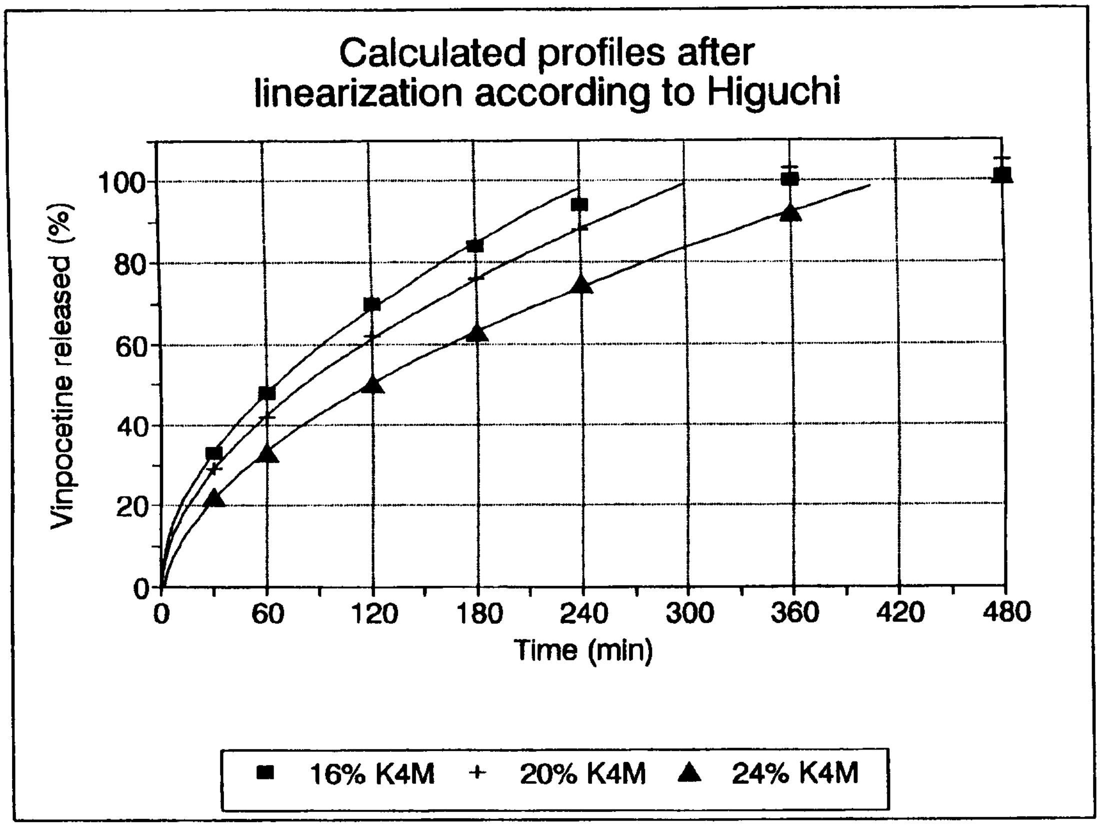


Fig. 3

European Patent Office

# EUROPEAN SEARCH REPORT

Application Number
EP 94 67 0004

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | WO-A-90 02141 (AUSTRALIAN COMMERCIAL RESEARCH & DEVELOPMENT LTD.)<br>* page 12, line 21 - page 14, line 16; table 1 *<br>* page 23, line 17 *<br>* page 26 - page 27; claims *<br>--- | 1-15 | A61K47/48 |
| X | PHARMAZIE,<br>vol.41, no.2, 1986, BERLIN DD<br>pages 151 - 152<br>M. KATA ET AL. 'ENHANCEMENT OF SOLUBILTY OF VINPOCETINE BASE WITH GAMMA-CYCLODEXTRIN.'<br>* the whole document *<br>--- | 1,4,6,7, 12-15 | |
| X | PHARMAZIE,<br>vol.37, no.5, 1982, BERLIN DD<br>pages 386 - 387<br>M. KATA ET AL. 'SPRÜHEINBETTUNG VON VINPOCETIN MIT BETA-CYCLODEXTRIN.'<br>* the whole document *<br>--- | 1,3,6,7, 12-15 | TECHNICAL FIELDS SEARCHED (Int.Cl.6) |
| X | CHEMICAL ABSTRACTS, vol. 103, no. 8, 26 August 1985, Columbus, Ohio, US;<br>abstract no. 59236,<br>* abstract *<br>& ACTA CHIM. HUNG.,<br>vol.118, no.2, 1985<br>pages 171 - 178<br>M. KATA ET AL. 'SPRAY PROCESSES IN DRUG RESEARCH'<br>---<br>-/-- | 1,7,8,12 | A61K |

The present search report has been drawn up for all claims

| Place of search | Date of completion of the search | Examiner |
|---|---|---|
| THE HAGUE | 7 September 1994 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 67 0004

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | PHARM. IND.,<br>vol.49, no.1, 1987<br>pages 98 - 100<br>M. KATA ET AL. 'INCREASING THE SOLUBILTY CHARACTERISTICS OF DRUGS WITH CYCLODEXTRINS.'<br>* the whole document *<br>----- | 1-15 | |
| | | | TECHNICAL FIELDS SEARCHED (Int.Cl.6) |

The present search report has been drawn up for all claims

| Place of search | Date of completion of the search | Examiner |
|---|---|---|
| THE HAGUE | 7 September 1994 | Berte, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)